# DEMANDE DE BREVET EUROPEEN

(11) **EP 1 707 889 A1**
(43) Date de publication de la demande: **04.10.2006**
(21) Numéro de dépôt: 06356032.0
(22) Date de dépôt: 21.03.2006
(51) Int. Cl.: F24F 6/12, A61L 9/04

(54) **Humidificateur d'air**

(30) Priorité: 29.03.2005 FR 0503037
(71) Demandeur: SEB SA, 69130 Ecully (FR)
(72) Inventeur: Serres Vives, Gérald, 01200 Eloise (FR); de Wasseige, Patrick, 74150 Moye (FR)
(74) Mandataire: Kiehl, Hubert

(57) **Abrégé**

Humidificateur d'air comportant, à l'intérieur d'un boîtier (1), un réservoir d'eau (5), un transducteur ultrasonore (25) qui met en vibration l'eau du réservoir et génère un flux de vapeur dans une enceinte (14) interne communiquant avec une ouverture (17) du boîtier, ainsi qu'un embout (4) de diffusion placé sur ladite ouverture (17) et présentant un orifice (20) de sortie de vapeur.

Selon l'invention, ledit embout (4) comporte une pièce interne (30) de forme allongée, réalisée en un matériau poreux apte à être imprégné de substance odorante volatile, qui est agencée de manière à permettre l'écoulement du flux de vapeur le long de ses parois avant d'atteindre ledit orifice (20) de sortie.

## Description

La présente invention concerne un appareil de traitement de l'air, notamment un humidificateur d'air apte à augmenter le taux d'hygrométrie d'une pièce ou d'un habitacle dans le but d'améliorer le confort respiratoire d'un individu qui s'y trouve.

Les humidificateurs les plus connus sont du type à générateur de vapeur chaude. Un tel appareil comporte généralement, à l'intérieur d'un boîtier, une cuve comportant de l'eau en contact thermique avec un élément chauffant qui évapore l'eau de la cuve et l'envoie, à travers une buse, à l'extérieur du boîtier dans la pièce à traiter. Afin de mieux combattre les problèmes respiratoires et de qualité de l'air, il a été souhaité d'élargir la sphère d'utilisation de tels appareils, notamment dans l'aromathérapie.

Ainsi, le document FR 1 346 401 décrit un appareil d'aromathérapie où l'eau présente dans une cuve est chauffée par un élément chauffant immergé au fond de la cuve et la vapeur est ensuite évacuée par une tête de pulvérisation dont l'extrémité inférieure traverse le couvercle de la cuve et l'extrémité supérieure forme une buse de sortie de vapeur. La tête de pulvérisation comporte par ailleurs, avant sa buse de sortie, un réservoir contenant des plantes imbibées de substances aromatiques. La vapeur sortant de la cuve entraîne les substances aromatiques à l'extérieur de l'appareil et elles se répandent ainsi dans la pièce. Fonctionnant comme humidificateur et appareil d'aromathérapie simultanément, cet appareil trouve vite ses limites car : il réchauffe la pièce en la rendant humide, il est bruyant et, de surcroît, il peut même s'avérer dangereux lorsqu'il est utilisé dans une chambre d'enfant.

On connaît également un autre type d'humidificateur d'air qui produit de la vapeur froide. Un tel appareil comprend généralement une réserve d'eau et un transducteur ultrasonore qui met en vibration la surface libre de l'eau et génère des gouttelettes d'eau dans un espace interne de l'appareil. Il comporte par ailleurs un embout distributeur qui oriente les gouttelettes d'eau vers l'extérieur de l'appareil, dans une pièce ou un habitacle. Un tel humidificateur trouve son usage optimal dans une chambre d'enfant en améliorant le confort respiratoire de celui-ci. Fonctionnant à satisfaction, cet appareil trouve ses limites lorsque l'on veut l'utiliser pour diffuser des substances aromatiques dans une chambre d'enfant, notamment des huiles essentielles dont l'action est bénéfique pour la santé des voies respiratoires. En effet, lorsqu'elles sont mélangées à l'eau du réservoir de l'appareil, les huiles essentielles s'avèrent être très agressives pour les pièces réalisées généralement en matière plastique de ce dernier et peuvent mettre en cause le bon fonctionnement de l'appareil.

Par ailleurs, le document JP2002-119584 décrit un humidificateur ultrasonore apte à diffuser un arôme dans une pièce. L'appareil comprend, dans un boîtier, un réservoir d'eau et un transducteur ultrasonore placé au fond du réservoir pour générer des vibrations dans l'eau et, par la suite, une brume de particules d'eau dans une enceinte en partie inférieure de l'appareil. L'appareil comprend, de plus, un diffuseur de parfum agencé à l'intérieur de son boîtier au dessus de l'enceinte contenant de la vapeur froide. Le diffuseur de parfum comprend une résistance électrique qui chauffe une tablette de substance aromatique afin de dégager des vapeurs de parfum dans une enceinte supérieure du boîtier. Un ventilateur prévu dans le boîtier de l'appareil produit un flux d'air qui pousse les particules d'eau de l'enceinte inférieure dans l'enceinte supérieure où le flux d'air les mélange aux vapeurs de parfum avant de les faire sortir dans la pièce par des ouvertures pratiquées dans la partie supérieure du boîtier de l'appareil. Un tel appareil arrive, certes, à évacuer de manière efficace dans une pièce des vapeurs humides chargées de molécules odorantes, mais, présente comme inconvénient principal le besoin de chauffer la substance aromatique. Or, il est connu que les propriétés thérapeutiques de l'huile diminuent lorsque l'huile est chauffée ou brûlée. Par ailleurs, l'utilisation d'un ventilateur rend l'appareil bruyant et complique sa construction.

Le but de la présente invention est de remédier aux inconvénients précités et de proposer un humidificateur d'air apte à améliorer le confort respiratoire et à assainir l'air, tout en étant d'un usage sécuritaire dans une chambre d'enfant.

Un autre but de l'invention est un humidificateur d'air apte à diffuser des substances aromatiques, notamment des huiles essentielles, en même temps qu'il humidifie une pièce, qui soit de construction simple et silencieux, tout en étant fiable en fonctionnement.

Un autre but de l'invention est de réaliser de manière simple et peu coûteuse un humidificateur d'air apte à diffuser des huiles essentielles qui soit sûr, efficace en fonctionnement et qui présente une durée de vie améliorée.

Ces buts sont atteints avec un humidificateur d'air comportant, à l'intérieur d'un boîtier, un réservoir d'eau, un transducteur ultrasonore qui met en vibration l'eau du réservoir et génère un flux de vapeur dans une enceinte interne communiquant avec une ouverture du boîtier, ainsi qu'un embout de diffusion placé sur ladite ouverture et présentant un orifice de sortie de vapeur, du fait que ledit embout comporte une pièce interne de forme allongée, réalisée en un matériau poreux apte à être imprégné de substance odorante volatile, qui est agencée de manière à permettre l'écoulement du flux de vapeur le long de ses parois avant d'atteindre ledit orifice de sortie.

Un tel humidificateur comprend un transducteur ultrasonore électronique qui génère des ondes ultrasonores dans le réservoir ou dans une cuve adjacente alimentée en eau par le réservoir de l'appareil et forme des fines gouttelettes à la surface libre de l'eau. Les gouttelettes d'eau ainsi générées forment un flux de vapeur froide, dans une enceinte interne de l'appareil, qui est ensuite évacué par l'orifice de sortie de l'embout de diffusion.

Selon l'invention, l'embout de diffusion de l'appareil comporte une pièce interne réalisée au moins en partie en un matériau poreux, agencée dans le flux de vapeur froide généré, avant son orifice de sortie. Ladite pièce interne forme ainsi une réserve de substance odorante volatile lorsqu'elle est chargée, par exemple en huile(s) essentielle(s), et présente une forme allongée orientée selon la direction du flux de vapeur de l'appareil, un ou plusieurs passages étant aménagés entre cette pièce et les parois internes de l'embout ou d'un conduit interne de l'appareil l'entourant. Lors des tests effectués en laboratoire, il a été constaté que, dans une telle configuration longiligne, les gouttelettes d'eau du flux de vapeur froide, lorsqu'elles sont forcées à longer, sur une certaine distance, les parois poreuses de la pièce interne de l'embout, elles forment des vecteurs de transport des particules volatiles de la substance odorante volatile qu'elles entraînent vers la sortie. Ces particules volatiles se mélangent au flux de vapeur de sortie et sont diffusées dans la pièce en même temps que ce dernier.

De surcroît, ladite pièce interne forme une réserve de substance aromatique volatile en étant réalisée au moins en partie en un matériau poreux, par exemple une éponge ou une pierre poreuse, ses alvéoles constituent des microréservoirs suspendus agencés dans le flux de vapeur, selon la direction de propagation de celui-ci, assurant ainsi une grande surface d'échange avec les gouttelettes d'eau et donc une diffusion uniforme des particules volatiles notamment d'huile essentielle.

Par substance odorante volatile on comprend une substance composée de molécules volatiles utilisée pour ses propriétés aromatiques, cette substance pouvant être une huile essentielle, une essence, voire même un parfum composé d'ingrédients de synthèse. On préfère toutefois utiliser l'appareil de l'invention avec des substances aromatiques naturelles, telles une huile essentielle (obtenue par distillation à la vapeur d'eau) ou une essence (obtenue par expression), voire un mélange d'huiles essentielles et/ou d'essences.

Un avantage important offert par cette solution est que la réserve d'huiles essentielles ou d'essences est supportée par l'embout de diffusion en étant donc suspendue dans le flux de vapeur et de ce fait elle ne touche pas les composants fonctionnels de l'appareil qui ne risquent alors pas d'être endommagés. Les particules d'huiles essentielles ne viennent en contact qu'avec un support fixé à l'embout diffuseur qui peut alors être réalisé en un matériau plus résistant, par exemple du polypropylène. Dans une variante, l'embout diffuseur peut être réalisé en plusieurs pièces montées amovibles, ledit support pouvant alors être retiré et remplacé en cas d'usure. Dans une autre variante, ledit support peut être placé sur l'ouverture du boîtier qui est alors entourée par un simple embout diffuseur permettant le passage de la vapeur vers l'extérieur de l'appareil.

Cet appareil offre une solution simple et efficace pour la diffusion d'huiles essentielles et/ou d'essences en même temps qu'il augmente le degré d'humidité d'une pièce. En étant transportées dans la pièce par les gouttelettes d'eau, les particules d'huiles essentielles et/ou d'essences gardent toutes leurs propriétés, elles désinfectent, désodorisent et parfument agréablement l'air dans la pièce. A titre d'exemple, les huiles essentielles ou essences qui débarrassent l'air ambiant des germes pathogènes et préviennent les affections microbiennes sont les huiles essentielles de citron, de thym, d'orange, de bergamote, de genièvre, de girofle, etc. seules ou en combinaison.

Avantageusement, ladite pièce interne est cylindrique et plonge à l'intérieur d'une enceinte tubulaire verticale.

Lorsqu'il est produit dans une enceinte verticale tubulaire, le flux de vapeur froide remonte vers un embout diffuseur disposé dans une ouverture de la partie supérieure du boîtier. Avant d'atteindre l'orifice de sortie du boîtier, le flux de vapeur froide longe les parois externes d'une pièce interne cylindrique qui présente alors la meilleure surface d'échange avec une enceinte tubulaire.

De préférence, le rapport entre le diamètre interne de l'enceinte tubulaire et le diamètre externe de ladite pièce interne est compris entre 1,1 et 2.

Lors des tests effectués en laboratoire, il s'est avéré qu'un tel rapport assurait une bonne surface d'échange, tout en permettant la circulation du flux de vapeur entre les deux pièces, ladite pièce interne étant de forme allongée (on comprend que sa longueur est supérieure à son diamètre).

Avantageusement, ladite pièce interne est un fourreau en mousse habillant un support.

Un tel fourreau en mousse est préféré car les huiles essentielles ou essences qu'il contient restent en surface. Le fourreau enveloppe le support sur tous ses côtés, augmentant ainsi la surface d'échange avec le flux de vapeur.

Dans une variante, la paroi de fond dudit fourreau est perméable au flux de vapeur de l'enceinte et ledit support est creux.

Ceci permet au flux de vapeur de lécher non seulement les parois externes du fourreau, mais également ses parois internes, pour une surface d'échange optimisée, avant de sortir en partie supérieure de l'embout.

De préférence, ledit embout et sa pièce interne sont montés amovibles par rapport au boîtier.

Ceci permet à l'utilisateur de charger à nouveau en substance aromatique le matériau poreux de l'embout, d'échanger les différents embouts entre eux, pour remplacer une substance aromatique par une autre, de le nettoyer ou encore de remplacer un embout usé ou un embout diffuseur de substance aromatique volatile par un embout simple qui ne diffuse que de la vapeur.

Avantageusement, l'humidificateur de l'invention comprend des moyens de réglage du débit de vapeur.

Ceci permet de régler la quantité d'huile essentielle diffusée dans la pièce en même temps que le taux d'humidité dans la pièce.

L'invention sera mieux comprise à l'étude d'un mode de réalisation pris à titre nullement limitatif et illustré dans les figures annexées dans lesquelles :
- la figure 1 représente une vue en coupe verticale d'un humidificateur d'air de l'invention;
- la figure 2 est une vue en perspective d'un embout diffuseur utilisé avec l'humidificateur de l'invention, le fourreau en matériau poreux étant enlevé pour plus de clarté.

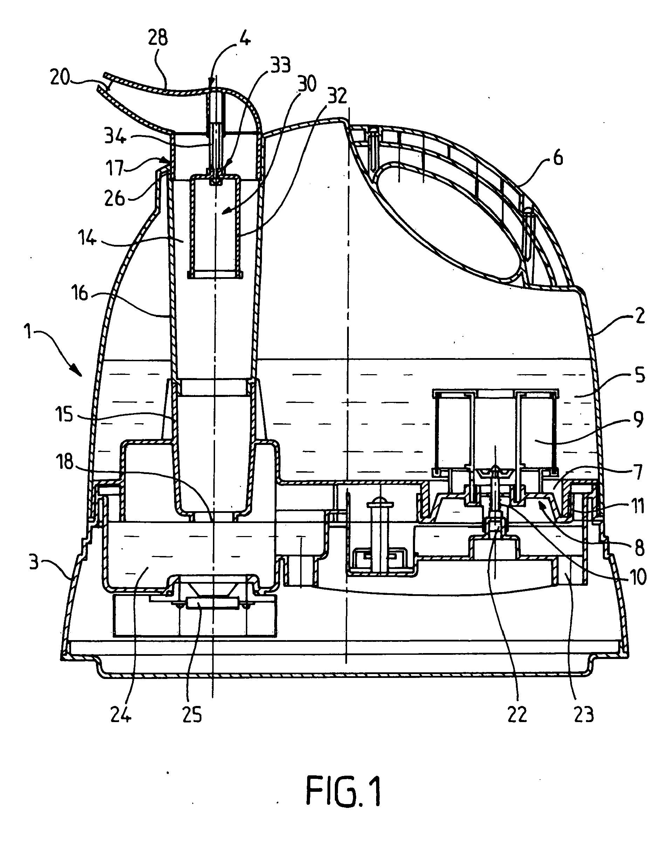

L'humidificateur d'air représenté à titre d'exemple à la figure 1 comprend un boîtier 1 en deux parties, notamment un corps creux 2 supporté par une base 3. Le corps creux 2 est monté amovible sur la base 3, il contient une réserve d'eau et présente une sortie de vapeur. La base 3 comporte, elle, des moyens d'alimentation en électricité des moyens électroniques de commande de l'appareil (non représentés sur la figure) et des moyens de transformation en vapeur de l'eau contenue à l'intérieur du corps creux 2, tel qu'il sera expliqué par la suite.

Le corps creux 2 est réalisé en un matériau plastique, de préférence transparent, et forme un réservoir d'eau 5. Il est muni d'une poignée 6 de préhension en sa partie supérieure et, en sa partie inférieure, d'un orifice de remplissage 7 obturé par un bouchon 8. Dans l'exemple représenté, le bouchon 8 comprend une cartouche anti-calcaire 9 et une soupape 10 qui traverse un couvercle 11 fileté autour des parois de l'orifice de remplissage 7. Lorsque l'on veut remplir en eau le réservoir 5 ou le vider, on le soulève de la base 3 en le prenant par la poignée 6, on le retourne et on dévisse le couvercle 11 pour enlever le bouchon 8. La cartouche anti-calcaire 9 peut être remplacée lors d'une telle opération, en la dévissant du bouchon 8. Le bouchon 8 est ensuite replacé sur le corps creux 2 et celui-ci peut reprendre sa place sur la base 3.

Le corps creux 2 comprend également une enceinte 14 tubulaire réalisée par deux tubes 15, 16 cylindriques ou légèrement coniques emboîtés et agencés de manière étanche à l'intérieur du réservoir d'eau 5. Les tubes 15, 16 délimitent l'enceinte 14 tubulaire et font communiquer, par une ouverture d'entrée 18 inférieure, la base 3 avec une ouverture 17 du boîtier réalisée en la partie supérieure du corps creux 2. L'ouverture 17 du boîtier est obturée par un embout 4 de diffusion monté pivotant dans un épaulement 26 de l'enceinte 14, situé en bordure de l'ouverture 17. L'embout 4 comporte, à son extrémité la plus éloignée du boîtier, un orifice de sortie 20 de vapeur.

La base 3 comprend une protubérance 22 qui prend appui sur la tête de la soupape 10 lorsque le corps creux 2 est posé sur la base 3. La protubérance 22 pousse alors la soupape vers sa position d'ouverture permettant à l'eau du réservoir 5 d'arriver, par un conduit 23 (représenté seulement en partie sur la vue en coupe partielle de la base de l'appareil) dans une cuve 24. Au fond de la cuve 24 est installé un transducteur ultrasonore 25, par exemple un transducteur piézoélectrique commandé par des moyens électroniques de commande (non représentés sur le dessin). Lorsque l'appareil est mis en marche, le transducteur ultrasonore 25 commence à vibrer à une fréquence prédéterminée et met en vibration le volume d'eau dans la cuve 24, ce qui provoque la désolidarisation des molécules d'eau formant alors une brume fine de gouttelettes d'eau au-dessus de la surface libre de l'eau dans la cuve 24. Les gouttelettes d'eau pénètrent par l'ouverture d'entrée 18 à l'intérieur de l'enceinte 14 formant un flux de vapeur froide qui est évacué à l'extérieur du boîtier par l'orifice de sortie 20 de l'embout 4 de diffusion.

Selon l'invention, l'embout 4 de diffusion comporte une pièce interne 30 réalisée en un matériau poreux contenant au moins une substance odorante volatile, de préférence une huile essentielle. La pièce interne 30 présente une forme allongée et, dans l'exemple représenté aux figures, elle comprend un support 33 creux fixé au corps de l'embout 4 et recouvert d'un fourreau 32 en mousse.

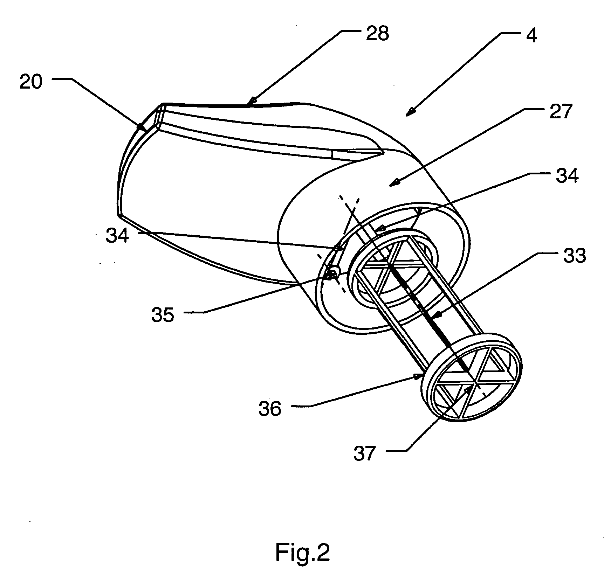

Tel que mieux visible en figure 2, l'embout 4 comprend un goulot 27 annulaire vertical destiné à prendre appui sur l'épaulement 26 de l'enceinte 14. Le goulot 27 se prolonge latéralement par un bec 28 dont les parois sont inclinées par rapport à l'axe vertical de l'enceinte 14 et forment une cheminée pour le flux de vapeur en provenance de l'enceinte, flux de vapeur qu'elles orientent ensuite vers l'orifice 20 de sortie. Le support 33 a une forme de cadre cylindrique ouvert et est réalisé à base de plusieurs croisillons reliés ensemble. Le support 33 est relié à l'embout 4 par une pièce en té 34 dont la base est attachée au centre de la partie supérieure du support 33 et dont les bras comportent chacun un ergot 35 s'insérant dans un orifice correspondant pratiqué à l'intérieur du corps de l'embout 4.

La surface latérale 36 du support 33 est recouverte d'un fourreau en mousse 32 (fig. 1) qui est un tube cylindrique déformable, de faible épaisseur (de l'ordre de quelques mm, enfilé de force sur le support 33. Dans une variante, le fourreau 32 comporte de plus une base qui recouvre également la paroi de fond 37 du support. Le fourreau 32 est imprégné d'huile essentielle une fois installé sur son support et l'embout est placé sur l'appareil.

En fonctionnement, lorsque l'appareil est mis en marche, des gouttelettes d'eau se forment au dessus de la cuve 24 et un flux de vapeur se crée à l'intérieur de l'enceinte 14. Lorsque le flux de vapeur arrive en la partie inférieure de la pièce interne 30 de l'embout 4, il est obligé d'emprunter le passage existant entre cette dernière et les parois de l'enceinte 4 ou alors il passe à l'intérieur du fourreau 32 en léchant ses parois. Lors de leur cheminement ascendant, les gouttelettes d'eau agissent en tant que vecteurs de transport des particules volatiles d'huile essentielle situées à la surface du fourreau 32. Ces particules se mélangent ainsi au flux de vapeur avant d'atteindre l'orifice de sortie 20 de l'embout 4. La pièce se trouve chargée en humidité et en même temps en vapeurs d'huiles essentielles.

D'autres variantes et modes de réalisation de l'invention peuvent être envisagés, sans sortir du cadre de ces revendications.

Ainsi, le boîtier de l'appareil peut être réalisé en une seule pièce et/ou comporter un seul réservoir, le transducteur piézoélectrique étant disposé au fond de ce réservoir.

Par ailleurs, la pièce interne de l'embout de diffusion peut être une pièce monobloc réalisée en un matériau poreux, telle une pierre poreuse ou une mousse pouvant être imprégnées d'huiles essentielles.

## Revendications

1. Humidificateur d'air comportant, à l'intérieur d'un boîtier (1), un réservoir d'eau (5), un transducteur ultrasonore (25) qui met en vibration l'eau du réservoir et génère un flux de vapeur dans une enceinte (14) interne communiquant avec une ouverture (17) du boîtier, ainsi qu'un embout (4) de diffusion placé sur ladite ouverture (17) et présentant un orifice (20) de sortie de vapeur, **caractérisé en ce que** ledit embout (4) comporte une pièce interne (30) de forme allongée, réalisée en un matériau poreux apte à être imprégné de substance odorante volatile, qui est agencée de manière à permettre l'écoulement du flux de vapeur le long de ses parois avant d'atteindre ledit orifice (20) de sortie.

2. Humidificateur d'air selon la revendication 1, **caractérisé en ce que** ladite pièce interne (30) est cylindrique et plonge à l'intérieur d'une enceinte (14) tubulaire verticale.

3. Humidificateur selon la revendication 2, **caractérisé en ce que** le rapport entre le diamètre interne de l'enceinte (14) tubulaire et le diamètre externe de ladite pièce interne(30) est compris entre 1,1 et 2.

4. Humidificateur d'air selon l'une des revendications précédentes, **caractérisé en ce que** ladite pièce interne (30) est un fourreau (32) en mousse habillant un support (33).

5. Humidificateur d'air selon la revendication 4, **caractérisé en ce que** la paroi de fond dudit fourreau est perméable au flux de vapeur de l'enceinte et que ledit support (33) est creux.

6. Humidificateur d'air selon l'une des revendications précédentes, **caractérisé en ce que** ledit embout (4) et sa pièce interne (30) sont montés amovibles par rapport au boîtier.

7. Humidificateur d'air selon l'une des revendications précédentes, **caractérisé en ce qu'**il comprend des moyens de réglage du débit de vapeur.
